# EUROPEAN PATENT SPECIFICATION

(11) **EP 2 876 169 B1**
(45) Date of publication and mention of the grant of the patent: **29.06.2016**
(21) Application number: 13194332.6
(22) Date of filing: 25.11.2013
(51) Int. Cl.: C12R 1/865, A23L 2/38, C12G 3/02, C12R 1/02, C12R 1/225, A23F 3/10, C12R 1/01, A23L 33/135

(54) **Association LMKK P1401 and method for obtaining fermented non-alcoholic polyfunctional synbiotic beverages**
Assoziation-LMK P1401 und Verfahren zur Herstellung fermentierter nichtalkoholischer polyfunktioneller synbiotischer Getränke
Association LMKK P1401 et procédé pour obtenir des boissons symbiotiques polyfonctionnelles non alcooliques fermentées

(43) Date of publication of application: 27.05.2015
(73) Proprietor: Latvijas Universitate, 1586 Riga (LV)
(72) Inventor: Semjonovs, Pavels, 1024 Riga (LV); Danilevics, Aleksejs, 1079 Riga (LV); Auzina, Lilija, 1055 Riga (LV); Denina, Ilze, 4401 Gulbene (LV); Upitis, Andris, 5000 Ogre (LV)
(74) Representative: Fortuna, Aleksandra

(56) References cited:
- DE-A1- 19 756 897
- DE-U1- 29 713 010
- DATABASE FSTA [Online] INTERNATIONAL FOOD INFORMATION SERVICE (IFIS), FRANkFURT-MAIN, DE; 2007, BADER J ET AL: "Controlled coculture fermentation for the production of new beverages.", XP002722650, Database accession no. FS-2008-07-Ha2699 & BREWING SCIENCE, no. Sept./Oct., 2007, page 128, BREWING SCIENCE

## Description

### Field of invention

The invention relates to food biotechnology and can be used for the obtaining of refreshing fermented beverages.

### Background Art

There are known natural microorganism associations that are used for obtaining of fermented beverages. For instance Tea fungus or Kombucha, Waterkefir or Tibi, Tibetan milk mushroom and kefir zoogloeas belong to such associations.

J ET AL: "Controlled coculture fermentation for the production of new beverages.",XP002722650,Database accession no. FS-2008-07-Ha2699 ; & BREWING SCIENCE,no. Sept./Oct., 2007, page 128, discloses a controlled coculture fermentation for the production of new beverages. The coculture composition comprises a combination of special strains of *Glucollobacter, Lactobacillus* and *Kluyveromyces*.

Zoogloeas of these microbial associations consist of various microorganisms, mainly different lactic acid and acetic acid bacteria and yeasts. For example in literature [1] is given such composition of Tea fungus natural microorganism association: acetic acid bacteria *Acetobacter xylinum, Acetobacter xylinoides* and yeasts *Schizosaccharomyces pombe, Saccharomyces ludwigii, Zygosaccharomyces rouxii, Candida sp., Pichia membrartefaciens.* According to another source [2] other yeast species were isolated from Tea fungus natural microorganism association - *Zygosaccharomyces bailii, Torulaspora delbreuckii, Rhodotorula mucilaginosa, Brettanomyces bruxellensis and Candida stellata.* Yeast *Saccharomyces cerevisiae* and lactic acid bacteria *Lactobacillus brevis* and *Streptococcus lactis* were isolated from Waterkefir association [3]. According to [4] lactic acid bacteria *Lactobacillus hordei, Lactobacillus nagelii, Lactobacillus casei, Leuconostoc mesenteroides, Lactobacillus citreum, Lactobacillus hilgardii* and yeasts *Saccharomyces cerevisiae, Lachancea fermentati, Hanseniaspora sp., Zygotorulaspora florentina* were found in this association.

Long since natural microorganism associations of zoogloeas have been used for the production of fermented beverages for household use. Fermentation liquids obtained by zoogloeas of natural microorganism associations have rich chemical composition. Zoogloeas of natural microorganism associations produce various biologically active organic acids, like acetic, lactic, succinic, gluconic, malic, citric acid and others during the fermentation. In addition mono and disaccharide residues remain in the liquid, small amount of ethanol is produced, vitamins and other biologically active substances are produced.

Whole complex of produced substances define biological value of the beverage and its organoleptic properties.

Use of natural microorganism associations' zoogloeas, their parts or parts of fermentation broth (as inoculums) is characteristic for current production of fermented refreshing beverages. Thus it is not possible to provide stability of constituents of microbial association, replicability and stability of technological process; it is not possible to warrant microbial safety of obtained beverage and its standardized quality; it is not possible to mechanize several stages of technological process that essentially raise the costs of the technological process and restricts production yield.

Therefore it is more convenient to use starter prepared beforehand with defined artificially selected microorganism composition not producing zoogloea for the production of beverage. It allows obtaining of healthy and tasty beverage during fast fermentation process. This kind of starter should also provide stable microbial composition and constant quality of various beverage batches.

### Disclosure of invention

Starter culture for obtaining fermented polyfunctional beverage - symbiotic association consisting of lactic acid and acetic acid bacteria and yeast was isolated from natural microbial association. Role of individual cultures of microbial association in development of properties of fermented beverage was evaluated during the selection of starter cultures. Microbiologically defined symbiotic lactic acid and acetic acid bacteria and yeast association and individual cultures of it do not form zoogloea that essentially simplify technological process and allows obtaining microbiologically safe beverages with standardized chemical composition and constant organoleptic properties. Use of the association allows essentially speed up fermentation process. Yeast isolates were identified in National Collection of Agricultural and Industrial Microorganisms, Faculty of Food Sciences, Corvinus University of Budapest. Isolates of bacteria were identified at Bacterial Collection of Microbiological Laboratory, Gent University (BCCM/LMG). Starter culture association that consists of individual cultures *Saccharomyces cerevisiae* LUMBI R46, *Gluconobacter nephelii* LUMBI B35 and *Lactobacillus sp.* LUMBI B90 in ratio 1:1:1 were deposited in Latvian Culture Collection of Microorganisms under accession number P 1401.

Constituent cultures of association LMKK P 1401 were identified as: (1) yeast *Saccharomyces cerevisiae* LUMBI R46; (2) acetic acid bacteria *Gluconobacter nepheli* LUMBI B35; (3) lactic acid bacteria *Lactobacillus sp.* LUMBI B90.
Individual microorganism cultures ratio in the association is 1:1:1.

Characterisation of the association LMKK P 1401 constituent cultures:

### Saccharomyces cerevisiae

White to cream, butyrous colonies. Vegetative reproduction by budding. Do not form filaments. Persistent asci containining up to 4 oval or round spores on McClary acetate agar at +25°C. Ferment D-glucose. Grow at +25 and +30° C, do not grow at +45°C. Do not grow in 60% glucose and 16% NaCl. Do not form starch, do not produce acetic acid, and do not hydrolyze urea. Do not ferment: lactose, cellobiose, D-xylose. Grow on: glucose. Do not grow on: L-sorbose, D-glucosamine, D-ribose, D-xylose, D-arabinose, L-arabinose, L-rhamnose, cellobiose, salicin, arbutin, lactose, inulin, erythritol, ribitol, xylitol, L-arabinitol, D-glucitol, D-mannitol, D-galactitol, myoinositol, D-glucono-1,5-D-lactone, 2-keto-D-gluconate, 5-keto-D-gluconate, D-gluconate, D-glucuronate, D-galacturonic acid, succinate, citrate, methanol, propane-1,2-diol, 2,3-butandiol, nitrites nitrates.

### Gluconobacter nephelii

Gram-negative rods (0.8-1 x 1.0-2.0 µm), motile. Grows at pH 3.0 +35°C. Optimum growth occurs at pH 5.5 and 25°C. Does not oxidize acetate or lactate. Grows on mannitol agar, does not grow on glutamate agar. Acid is produced from D and L-arabinose, meso-erytritol, ethanol, D-fructose, D-galactose, glucose, glycerol, maltose, D-mannitol, D-mannose, L-rhamnose, raffinose, D-sorbitol, sucrose, D-xylose. Acid is not produced from dulcitol, lactose, melibiose, L-sorbose. Grows on D un L-arabinose, meso-erytritol, fructose, glucose, glycerol, maltose, mannitol, raffinose, rhamnose, sorbitol, sorbose, sucrose, pentitols, D and L- arabitol. Does not grow on dulcitol, ethanol, galactose, lactose, D-mannose, melibiose, D- xylose.

### Lactobacillus sp

Cells are nonmotile rods, nonspore-forming, gram-positive. Fermentative metabolism, obligately saccharoclastic. At least half of the end product carbon is lactate. Facultatively anaerobic, do not reduce nitrates. Gelatine not liquefied. Indole and H₂S not produced. Catalase negative, cytochrome negative. Benziline reaction negative. Do not produce pigments. Colonies on MRS agar white, shiny. Optimum growth temperature +30°C.

### Example of implementation of the invention

### Example 1

Fermentation of sucrose solution 100 g/l supplemented with raisins 10 g/l is carried out in 400 ml glass containers. Freeze-dried biomass of individual cultures of association is used as inoculum (1.5 g/l). Bacteria and yeast ratio in the starter is 2:1. Fermentation is performed at +30° C, during 24 - 48 hours, no aeration or mixing was applied. Highest scores in organoleptic evaluation got the beverage after 24 h fermentation. Composition of fermentation medium and physically-chemical properties of fermented beverage is shown in Table 1. High concentration of malic acid and lactic acid should be pointed out. Association does not form zoogloea during the fermentation that essentially simplifies the application of association LMKK P 1401 for industrial fermentation.

**Table 1**

| Composition of fermented beverage and its physically-chemical properties | | |
|---|---|---|
| **Parameter** | **Parameter's indices** | |
| Fermentation medium | Sucrose, 100 g/l; raisins 10 g/l | |
| Composition of inoculum | Lactic acid bacteria LUMBI B90: acetic acid bacteria LUMBI B35: yeast LUMBI R46= 1:1:1 Amount of inoculum s 1.5g/l | |
| | Duration of fermentation, hours | |
| | **24** | **48** |
| Biomass g/l | 1.45 | 1.87 |
| Titratable acidity ⁰T | 58 | 123 |
| pH | 2.73 | 2.51 |
| Glucose,% | 2.69 | 1.67 |
| Fructose,% | 3.15 | 2.73 |
| Sucrose,% | 5.13 | 1.79 |
| Gluconic acid, | 0.25 | 0.73 |
| mg/ml | | |
| Fructans, mg/ml | - | 1.09 |
| Citric acid, mg/ml | 0.18 | 0.20 |
| L-malic acid, mg/ml | 2.2 | 1.67 |
| Succinic acid, mg/ml | 0.29 | 0.45 |
| Lactic acid, mg/ml | 3.47 | 4.04 |
| Acetic acid,mg/ml | 0.18 | 1.69 |
| Ethanol, mg/ml | 1.9 | 2.6 |

The offered three-component association produces wide spectra of organic acids. Lactic acid and malic acid were produced in relatively higher concentrations. Synthesis of fructans during fermentation was observed. Sensory properties of obtained beverage were evaluated as pleasant. Beverage after 24 hour fermentation was evaluated as better than beverage after 48 hour fermentation.

### Example 2

Fermentation medium consists of decocted black tea (4 g/l), sucrose 70 g/l; fermentation is carried out in 400 ml glass containers. Freeze-dried biomass of individual cultures of association is used as inoculum (1.5 g/l). Bacteria and yeast ratio in the starter is 2:1. Fermentation is performed at +30° C, during 24 - 48 hours, no aeration or mixing was applied. Composition of fermentation medium and physically-chemical properties of fermented beverage is shown in Table 2.

**Table 1**

| | | |
|---|---|---|
| Composition of fermented beverage and its physically-chemical properties | | |

| **Parameter** | **Parameter's indices** | |
|---|---|---|
| Fermentation medium | Tea 4 g/l, sucrose 7% | Tea 4 g/l, sucrose 7% |
| Composition of inoculum | Lactic acid bacteria LUMBI B90: acetic acid bacteria LUMBI B35: yeast LUMBI R46= 1:1:1 Amount of inoculum s 1.5g/l | Tea fungus natural microbial association |
| Duration of fermentation, hours | 48 | 48 |
| pH | 2.73 | 5.5 |
| Titratable acidity ⁰T | 120 | 12 |
| Reducing sugars, RV, % | 5.67 | 6.5 |
| Citric acid, mg/ml | 0.06 | 0.05 |
| Succinic acid, mg/ml | 0.40 | 0.09 |
| Lactic acid, mg/ml | 0.52 | - |
| Acetic acid,mg/ml | 0.74 | 0.04 |
| Gluconic acid, mg/ml | 3.78 | 0.05 |
| L-malic acid, mg/ml | 1.73 | 0.05 |
| Ethanol, mg/ml | 2.86 | 0.21 |
| Fructans | 1.52 | - |
| Formation of zoogloea | No | Yes |

The association LMKK P 1401 does not form zoogloea. Unlike tea fungus natural association, offered association contains lactic acid bacteria that enriches fermented beverage with lactic acid - valuable for human health and attributes to it potentially probiotic properties. Duration of fermentation by association LMKK P 1401 is substantially shorter (48 h) in comparison with natural association (7-14 days). Applying natural association, fermented beverage is not ready yet after 48 h and does not contain fructans (Table 2).

This beverage obtained by the three-component association contains relatively high concentration of gluconic acid (3.78 mg/ml) and malic acid (1.73 mg/ml). Beverage contains other organic acids e.g. citric, succinic, lactic and acetic acid.

### References

1. Chen C., Lin B.I. (2000) Changes in major components of tea fungus metabolites during prolonged fermentation. J Appl Microbiol, 89:834-839.
2. Teoh A.L, Heard G., Cox J. (2004) Yeast ecology of Kombucha fermentation. Int J Food Microbiol, 95:119-126.
3. Reiβ J. (1990) Metabolic activity of Tibi grains. Lebensm Unters Forsch, 191:462-465.
4. Gulitz A., Stadie I., Wenning M., Ehrman M.A., Vogel R.E. (2011) The microbial diversity of water kefir. Int J Food Microb, 151:284-288.

## Claims

1. An association of microbial cultures consisting of individual cultures *Lactobacillus sp.* LUMBI B90, *Gluconobacter nephelii* LUMBI B35 and *Saccharomyces cerevisiae* LUMBI R46 in ratio 1:1:1 deposited in Latvian Culture Collection of Microorganisms under accession number P 1401.

2. Use of the association according to claim 1 as a starter culture for the production of fermented beverages.

3. A method for obtaining fermented beverages using the association in accordance with claim 1, where fermentation is performed at +15+40 °C, fermentation medium contains carbohydrates or carbohydrates containing raw materials 1- 300 g/l.

4. The method according to claim 3, where medium containing carbohydrates or raw materials containing carbohydrates are malt, molasses, honey, fruit juice, vegetable juice, fruit juice concentrate or vegetable juice concentrate.

5. The method according to claim 3 or 4, where fermentation medium is supplemented with dried or fresh fruits, berries or vegetables, plant premixes, their concentrates or extracts before or after fermentation.

## Patentansprüche

1. Eine Sammlung mikrobieller Kulturen, die aus einzelnen Kulturen des *Lactobacillus sp.* LUMBI B90, *Gluconobacter nephelii* LUMBI B35 und *Saccharomyces cerevisiae* LUMBI R46 im Verhältnis 1:1:1 besteht und in einer lettischen Kultursammlung von Mikroorganismen unter der Zugangsnummer P 1401 aufbewahrt wird.

2. Verwendung der Sammlung gemäß Anspruch 1 als Starterkultur für die Herstellung fermentierter Getränke.

3. Verfahren zur Herstellung fermentierter Getränke, bei der die Sammlung gemäß Anspruch 1 verwendet und die Fermentierung bei + 15 + 40 ° C durchgeführt wird, und das Fermentationsmedium Kohlenhydrate oder rohstoffhaltige Kohlenhydrate in einer Menge von 1- 300 g/l enthält.

4. Verfahren nach Anspruch 3, bei dem das Medium, das Kohlenhydrate oder rohstoffhaltige Kohlenhydrate enthält aus Malz, Melasse, Honig, Fruchtsaft, Gemüsesaft, Fruchtsaftkonzentrat oder Gemüsesaftkonzentrat besteht.

5. Verfahren nach Anspruch 3 oder 4, in dem das Fermentationsmedium vor oder nach der Fermentierung durch getrocknete oder frische Früchte, Beeren oder Gemüse, Pflanzvormischungen, ihre Konzentrate oder Extrakte ergänzt wird.

## Revendications

1. Association de cultures microbiennes constituée de cultures individuelles *Lactobacillus sp.* LUMBI B90, *Gluconobacter nephelii* LUMBI B35 et *Saccharomyces cerevisiae* LUMBI R46 dans un rapport 1:1:1, déposées à la collection de cultures de micro-organismes lettone (Latvian Culture Collection of Microorganisms) sous le numéro d'accession P 1401.

2. Utilisation de l'association selon la revendication 1 en tant que culture de départ pour la production de boissons fermentées.

3. Procédé d'obtention de boissons fermentées en utilisant l'association selon la revendication 1, où la fermentation est effectuée à +15 à +40 °C, le milieu de fermentation contient des glucides ou des matières premières contenant des glucides à raison de 1 à 300 g/L.

4. Procédé selon la revendication 3, où le milieu contenant des glucides ou les matières premières contenant des glucides sont du malt, de la mélasse, du miel, du jus de fruits, du jus de légumes, un concentré de jus de fruits ou un concentré de jus de légumes.

5. Procédé selon la revendication 3 ou 4, où le milieu de fermentation est complété avec des fruits, baies ou légumes, séchés ou frais, des prémélanges de plantes, leurs concentrés ou extraits avant ou après fermentation.
